# EUROPÄISCHE PATENTSCHRIFT

(11) **EP 0 456 047 B1**
(45) Veröffentlichungstag und Bekanntmachung des Hinweises auf die Patenterteilung: **17.01.1996**
(21) Anmeldenummer: 91106742.9
(22) Anmeldetag: 26.04.1991
(51) Int. Cl.: C12Q 1/68

(54) **Verfahren zur Detektion und quantitativen Bestimmung von Nitrosomonas-Stämmen in Abwässern oder Boden**
Method for the detection and quantitative determination of nitrosomonas strains in waste water or soil
Méthode pour déceler et déterminer quantitativement des souches de nitrosomonas dans les eaux résiduaires ou dans le terrain

(30) Priorität: 09.05.1990 DE 4014845
(43) Veröffentlichungstag der Anmeldung: 13.11.1991
(73) Patentinhaber: BAYER AG, D-51368 Leverkusen (DE)
(72) Erfinder: Springer, Wolfgang, Dr., W-5600 Wuppertal 1 (DE); Rast, Hans Georg, Dr., W-5060 Bergisch Gladbach (DE); Löbberding, Antonius, Dr., W-5600 Wuppertal 1 (DE); Kanne, Reinhard, Dr., W-5090 Leverkusen (DE)

(56) Entgegenhaltungen:
- CANADIAN JOURNAL OF MICROBIOLOGY, Band 34, Nr. 10, Oktober 1988, Seiten 1122- 1128, CA; R.D. JONES et al.: "A new marine ammonium-oxidizing bacterium, Nitrosomonas crytolerans sp. nov."
- INTERNATIONAL JOURNAL OF SYSTEMATIC BACTERIOLOGY, Band 33, Nr. 3, July 1983, Seiten 521-524, International Union of Microbiological Societies; M.S.DODSON et al.;
- HAKKOKOGAKU KAISHI, Band 66, Nr. 2, 1988, Seiten 103-107; T. TOKUYAMA et al.: "Cloning and expression of the hydroxylamine oxidase gene of Nitrosomonas europaea in Pseudomonas putida"

## Beschreibung

Ammonium ist aufgrund seiner Fischtoxizität, insbesondere jedoch wegen seiner die Eutrophierung von Gewässern fördernden Eigenschaft ein Stoff, der möglichst vollständig aus Abwasser entfernt werden soll. Während für hohe Ammonium-Konzentrationen zahlreiche technische Verfahren existieren, ist die Entfemung von Ammonium im ppm-Bereich wirtschaftlich nur durch biologische Verfahren zu erreichen. Dies geschieht durch zwei spezialisierte Gruppen von Bakterien, die aus der Oxidation von Ammoniak zu Nitrit (Ammoniakoxidierer) und weiter zu Nitrat (Nitritoxidierer) ihre für den Zellstoffwechsel nötige Energie gewinnen. Als Kohlenstoffquelle verwenden die Ammoniakoxidierer ausschließlich CO₂, Nitritoxidierer verwerten zusätzlich auch andere Kohlenstoffquellen. Das entstandene Nitrat kann durch eine Vielzahl heterotropher Bakterien bei niedriger Sauerstoffkonzentration zu Stickstoff reduziert und damit vollständig entfernt werden (siehe z.B. (1)).

Nachteile bei der mikrobiellen Stickstoffeliminierung sind jedoch die geringen Wachstumsraten der Nitrifikanten. Als Kohlenstoffquelle wird ausschließlich CO₂ verwertet, was einen nur geringen Zellzuwachs bedingt. Zahlreiche organische Verbindungen wie Isothiocyanate, Amine, Phenole und stickstoffhaltige Heterocyclen hemmen darüber hinaus das Wachstum der Ammoniak oxidierenden Bakterien. Dies führt zu einer starken Einschränkung der biologischen Stickstoffelimination in industriellen Kläranlagen.

Da eine schnelle und quantitative Bestimmung von Nitrifikanten mit mikrobiologischen Kulturmethoden nicht möglich ist, ergaben sich bisher auch keine Möglichkeiten Veränderungen in der Nitrifikantenmenge von Abwasserpopulationen zu erkennen und durch entsprechende Steuerungsmaßnahmen eine optimale Klärleistung zu erreichen.

Dieses Problem konnte erfindungsgemäß dadurch gelöst werden, daß Gensonden für diese Ammoniak oxidierenden Bakterien konstruiert wurden. Mit diesen Gensonden ist es möglich, in komplexen Biomassen, wie sie in Abwässern und Böden vorliegen, eine quantitative Bestimmung dieser Nitrifikanten auf der Basis der Nukleinsäurekonzentration vorzunehmen. Bei dem Gensonden-Test kommt es zu einer spezifischen Duplexbildung von Gensonde und komplementärer Target-DNA aus Nitrifikanten (Hybridisierung). Die Gensonde wird auf der Basis der nachfolgend beschriebenen Sequenz entweder synthetisch (bis 100 Oligonukleotide) oder biologisch hergestellt und mit einer Markierung (Radioaktivität, Farbstoff, Enzym) versehen. Gibt man sie zu einem beliebigen Probenmaterial, das Nitrifikanten enthält, z.B. Nukleinsäurelysate aus Klärschlamm oder Böden, so kommt es aufgrund der komplementären Sequenzen von Gensonde und Nitrifikanten-DNA zur Hybridisierung. Diese Hybridisierungsreaktion kann in Lösung oder mit trägerfixierten Nukleinsäuren (Nitrozellulose-, Nylonmembranen, Beads) durchgeführt werden. Die hybridisierte Nukleinsäure wird über die Markierung der Gensonde quantitativ ausgewertet und gibt so ein direktes Maß für den Nitrifikantengehalt der Biomasse des Abwassers oder Bodens.

Hauptanwendungsgebiete für die neuen Gensonden sind die Überprüfung und Überwachung der Nitrifikantenkonzentration bei der biologischen Stickstoffeliminierung in Kläradagen.

Die Nachweisgrenze für die Detektion von Nitrifikanten mit den neuen Gensonden liegt im Bereich von 10⁵ bis 10⁶-Bakterien.

Die Nachweisempfindlichkeit kann durch Amplifikationsmethoden wie z.B. der PCR-Methode (Polymerase Chain Reaction) noch deutlich verbessert werden. Mit der Amplifikation auf der Basis der in der Erfindung beschriebenen Sequenzen der Gensonden kann so eine Detektion von 10 bis 100 Bakterien erreicht werden.

Im folgenden wird die Erfindung anhand von Ausführungsbeispielen näher erläutert.

### Gentechnologische Isolierung von Nitrosomonas-Gensonden

Die genomische Nukleinsäure aus dem Stamm Nitrosomas europaea ATCC 19718 wurde nach einem präparativen Lysozym/SDS-Nukleinsäure-Isolierungsverfahren isoliert.

Zur Konstruktion von Gensonden wurde die genomische DNA mit dem Restriktionsenzym BamHI in 1-15 kb große DNA-Fragrnente gespalten und mit BamHI linearisiertem pBR322 Plasmidvektor gentechnologisch verknüpft und in kompetente E.coli-Zellen AG1 transformiert.

Ampicillinresistente Klone wurden am nächsten Tag isoliert und rekombinante Klone, die DNA von Nitrosomonas enthielten, über die Tetrazyklinsensitivität des Plasmidvektors identifiziert. Die Klonierungsexperimente wurden mit der von Maniatis (2) beschriebenen Gen-Technik durchgeführt.

Nach einem analytischen Nukleinsäureisolierungsverfahren wurde die Plasmid-DNA aus den rekombinanten Klonen isoliert und durch gelelektrophoretische Auftrennung die Größe der eingebauten Nitrosomonas-DNA bestimmt. Die genaue Größe von Klonen mit eingebauter Nitrosomonas-DNA im Bereich von 1-15 kb wurde durch Spaltung der Plasmide mit dem Restriktionsenzym BamHI und gelelektrophoretischer Auftrennung der linearisierten Plasmide genau bestimmt. In der Tabelle 1 sind Klone mit den genauen Insertgrößen an Nitrosomonas-DNA aufgelistet

Durch Reversed Phase Hybridisierung wurde überprüft, welche der isolierten Nitrosomonas-Gensonden das breiteste Detektionsspektrum für Nitrosomonas aufweist Dazu wurden die einzelnen Gensonden gelelektrophoretisch im Agarosegel aufgetrennt und anschließend die Gensonden-DNA vom Gel auf Nitrocellulose-Filter übertragen und fixiert. Die genomische Nitrosomonas-DNA wurde mit einer geeigneten Markierung (P³-ATP, Biotin, Digoxigenin, Enzym) versehen und als Gensonde in dem Southern Blot mit den auf Nitrocellulose-Filter fixierten Gensonden eingesetzt.

Es wurde eine 6 kb große Gensonde SPN 323.13 identifiziert, die im Southern Blot-Hybridisierungstest ein sehr starkes Hybridisierungssignal ergab. Diese Gensonde wurde auf ihre Nitrosomonas-Spezifität durch einen DOT Blot-Hybridisierungstest analysiert. Die Experimente ergaben, daß die Gensonde SPN 323.13 spezifisch für Nitrosomonas ist und von allen Gensonden das breiteste Detektionsspektrum für Nitrosomonas aufwies. Diese Gensonde wurde für die weitere Entwicklung von kürzeren Gensonden und chemisch synthetisierten Oligonukleotid-Gensonden eingesetzt.

### Gentechnologische Entwicklung von Nitrosomonas-Gensonden

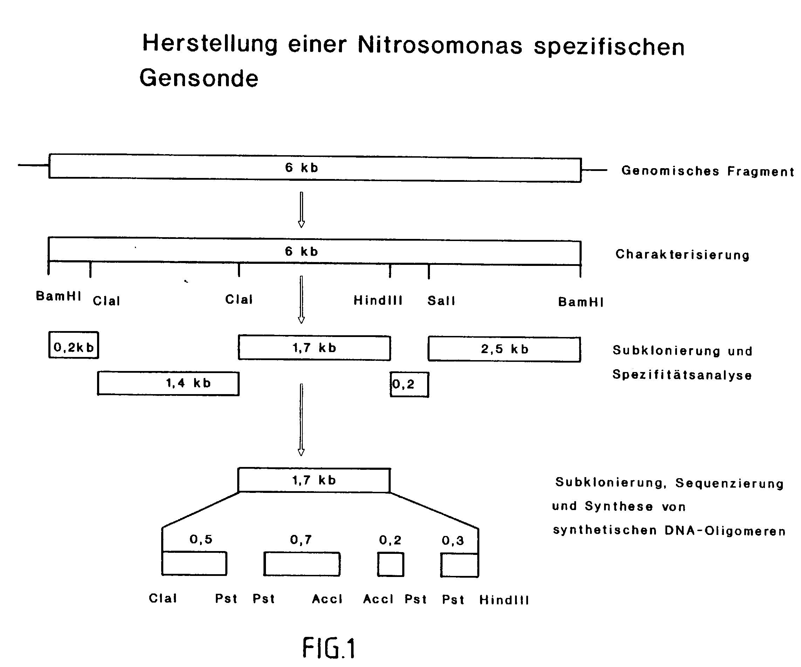

Auf der Basis der 6 kb großen Gensonde SPN 323.13 wurden weitere verkürzte Gensonden konstruiert (Abb. 1).

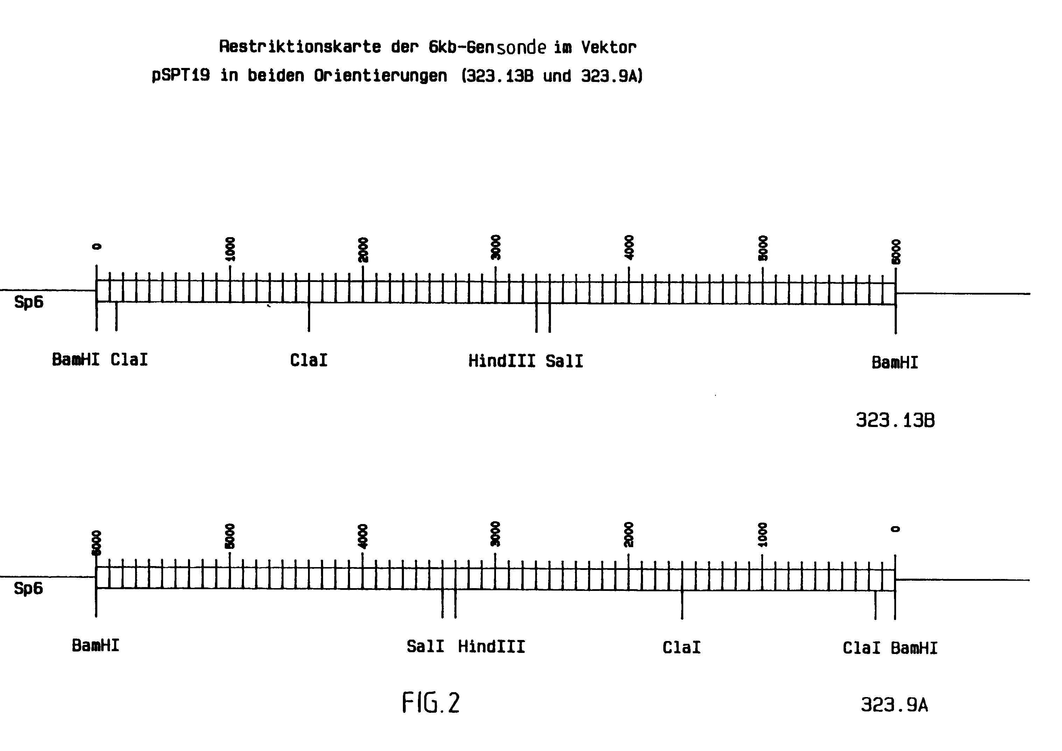

Die Gensonde wurde zunächst durch Spaltung mit verschiedenen Restriktionsenzymen molekularbiologisch charakterisiert Die lineare Restriktionskarte der Gensonde ist in beiden Orientierungen in Abbildung 2 dargestellt.

Das 1,4 kb große ClaI-Fragment, das 1,7 kb große ClaI-HindIII-Fragment und das 2,5 kb große SalI-BamHI-Fragment wurden in die Vektoren pBR322 und pSK Bluescript von Stratagene subkloniert.

Rekombinante Klone mit den einzelnen Genfragmenten wurden isoliert und molekularbiologisch charakterisiert. Die einzelnen Genfragmente wurden nach Spaltung mit den entsprechenden Restriktionsenzymen und gelelektrophoretischer Auftrennung durch Elektroelution isoliert. Diese Gensonden wurden dann mit den üblichen Markierungssubstanzen (P32-ATP, Biotin, Digoxigenin, Enzyme) markiert und im Gensonden-Test die Spezifität der Gensonden überprüft.

Aufgrund der hohen Spezifität für Nitrosomonas-Stämme und dem dennoch sehr breiten Detektionsspektrum für Nitrosomonas wurde die 1,7 kb große Gensonde SPN 366.1 als besonders geeignet für die Nitrosomonas-Detektion identifiziert.

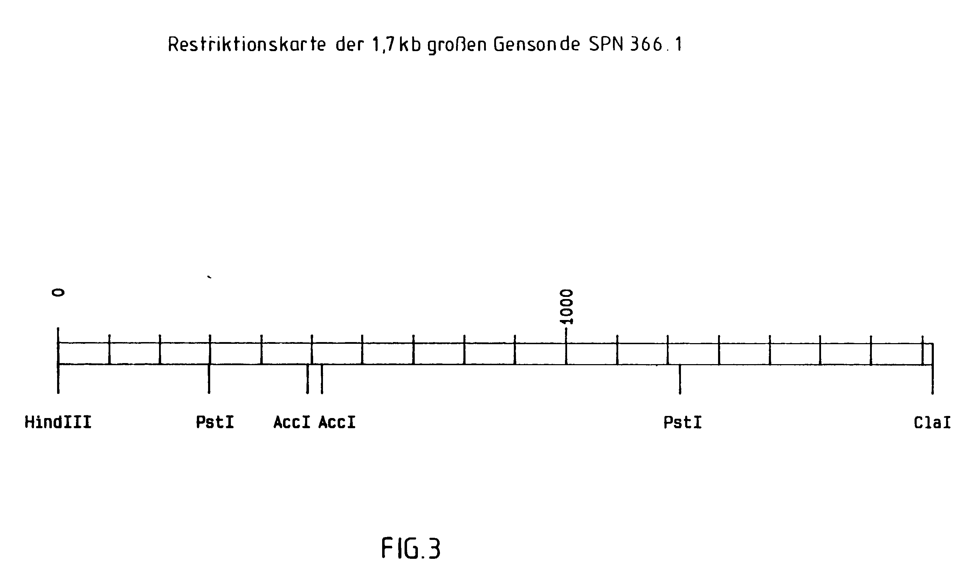

Diese Gensonde wurde zur weiteren Optimierung und Synthese von Oligonukleotid-Gensonden zunächst molekularbiologisch durch Spaltung mit Restriktionsenzymen charakterisiert. Die lineare Restriktionskarte der 1,7 kb-Gensonde SPN 366.1 ist in Abbildung 3 dargestellt.

Zur Verkürzung der 1,7 kb großen Gensonde SPN 366.1 wurden die 0,2 kb, 0,3 kb, 0,5 kb und 0,7 kb großen Genfragmente, die durch Spaltung mit den Restriktionenzymen HindIII, AccI, PstI und ClaI entstehen, in den Vektor pSK Bluescript® von der Firma Stratagene subkloniert.

Die 0,2 kb, 0,3 kb, 0,5 kb und 0,7 kb großen Gensonden wurden aus den entsprechenden Konstrukten nach Spaltung mit Restriktionsenzymen und gelelektrophoretischer Auftrennung im Agarosegel durch Elektroelution isoliert. Die Gensonden wurden mit einer Markierung (P³ATP, Biotin, Digoxigenin-UTP, Enzym) versehen nach der Methode des Random Prime Labelling und anschließend im Gensonden-Test auf ihre Spezifität und Empfindlichkeit untersucht Es wurde festgestellt, daß die Gensonden SPN 391.7 (0,2 kb), SPN 397.1 (0,3 kb), SPN 391.3 (0,5 kb) und SPN 391.16 (0,7 kb) keine signifikanten Unterschiede in Bezug auf die Spezifität und Sensitivität der Nitrosomonas-Detektion zeigten und alle gleichermaßen geeignet für den Einsatz als Gensonden sind. In Tabelle 2 sind die Gensondentests mit den Gensonden SPN 391.7, 397.1, 391.3 und 391.16 im Vergleich mit der 1,7 kb-Gensonde SPN 366.1 dargestellt.

### Sequenzierung der Gensonde SPN 366.1

Auf der Basis der Gensonde SPN 366.1 (1,7 kb) und den 0,2 kb, 0,3 kb, 0,5 kb und 0,7 kb großen Gensonden wurde die Sequenzierung der 1,7 kb-Gensonde durchgeführt.

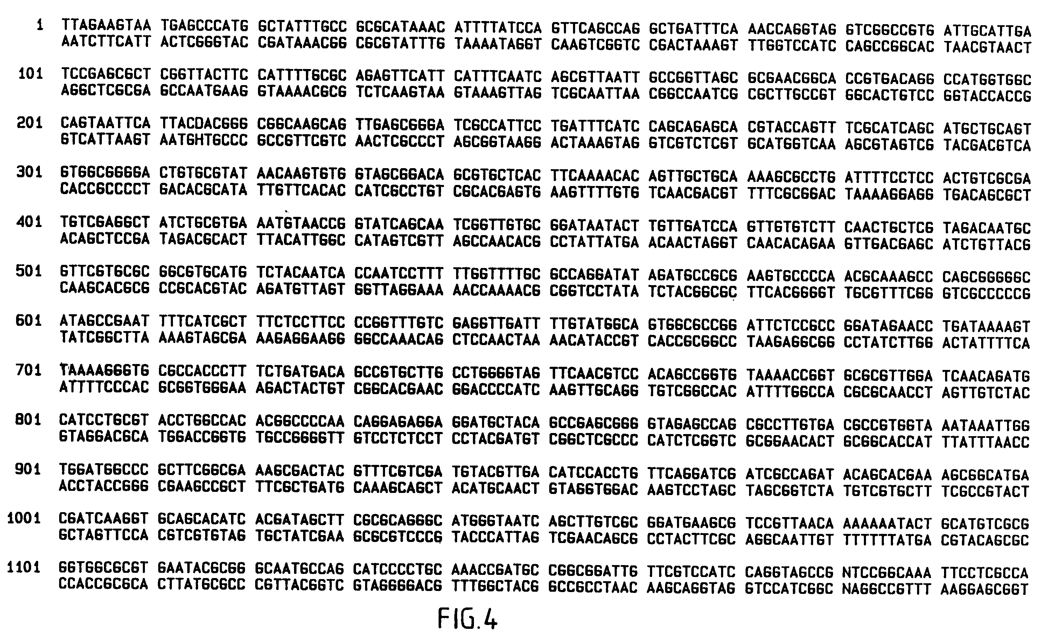

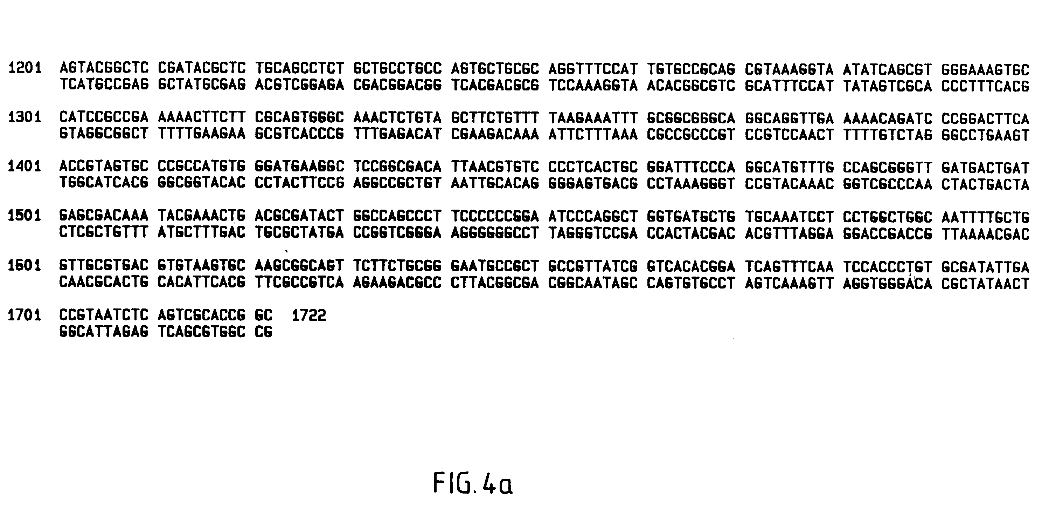

Die Sequenzierung wurde nach der Dideoxykettenabbruchmethode von Sanger (5) vorgenommen. Die Nukleotidsequenz der 1,7 kb-Gensonde SPN 366.1 ist in Abbildung 4 dargestellt.

### Chemische Synthese von Oligonukleotid-Gensonden

Die chemische Synthese von Oligonukleotid-Gensonden wurde auf der Basis der vorliegenden Sequenz der 1,7 kb-Gensonde SPN 366.1 nach der Amidit-Methode von S.L. Beaucage und M.H. Caruthers durchgeführt (6).

Durch Gensonden-Teste mit verschiedenen Oligonukleotiden aus dem Bereich der 1,7 kb-Gensequenz der Gensonde 366.1 wurde festgestellt, daß 15mer-100mer Oligonukleotid-Gensonden aus beliebigen Bereichen der 1,7 kb-Gensonde für den Gensonden-Test eingesetzt werden können.

Folgende Oligonukleotid-Gensonden erwiesen sich im Gensonden-Test als besonders geeignet:

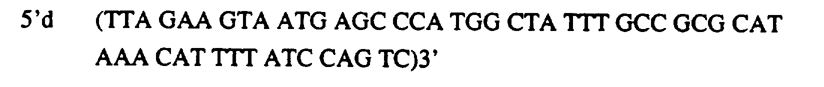

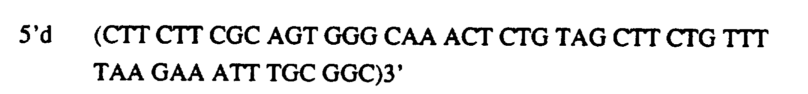

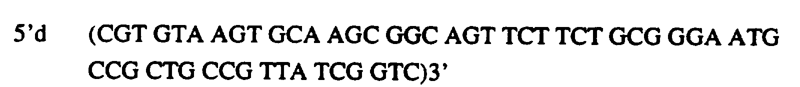

### Durchführung des Gensonden-Tests

Zur Überprüfung der Spezifität und Sensitivität der Gensonden wurde ein Gensonden-Test mit dem Digoxigenin-Test-Kit der Firma Boehringer, Mannheim mit Digoxigenin-UTP-markierten Gensonden durchgeführt.

### Markierung der Nitrosomonas-Gensonden

Die Gensonden wurden nach der Random-Prime-Methode von Feinberg und Vogelstein (3) mit Digoxigenin-UTP markiert. Vor der Random-Prime-Markierung wurden die Gensonden aus den entsprechenden rekombinanten Plasmiden mit Restriktionsenzymen herausgeschnitten. Die linearisierten Gensonden wurden gelelektrophoretisch im 0,8 %igen Agarosegel von dem linearisierten Plasmidvektor abgetrennt. Die Gensonden-DNA wurde aus dem Agarosegel herausgeschnitten und die Gensonde aus dem Agaroseblock durch Elektroelution isoliert. Die Gensonden-DNA wurde anschließend durch Phenolextraktion und Ethanolfällung weiter gereinigt. Vor der Markierung mit Digoxigenin-UTP wurde die Gensonden-DNA durch 10 min Erhitzen im Wasserbad auf 100°C und schnellem Abkühlen auf Eis/NaCl denaturiert. Zur Markierung wurden 1 µg denaturierte Gensonden-DNA, 2 µl Hexanukleotid-Gemisch, 2 µl dNTB-Markierungsgemisch zusammengegeben, mit sterilem bidest. Wasser auf 19 µl aufgefüllt und 1 µl Klenow-Enzym zugegeben. Die Markierung mit Digoxigenin-UTP wurde 60 min bei 37°C durchgeführt. Anschließend wurde die Reaktion durch Zugabe von 2 µl EDTA-Lösung 0,2 Mol/l pH 8 abgestoppt, die markierte DNA mit 2,5 µl LiCl 4 Mol/l und 75 µl vorgekühltem Ethanol (-20°C) präzipitiert. Nach 30 min bei -70°C wurde der DNA-Niederschlag bei 12 000 g abzentrifugiert und mit kaltem Ethanol, 70 %, gewaschen, im Vakuum getrocknet und in 50 µl Tris-HCl 10 mmol/l, EDTA 1 mmol/l pH 8 gelöst

### Hybridisierung mit Nitrosomonas-Gensonden

Für die Hybridisierungsexperimente wurden die Nitrosomonas-DNA enthaltenden Nukleinsäureextrakte aus der Biomasse von Abwässem oder Böden zunächst durch 10 min Erhitzen auf 100°C und schnellem Abkühlen auf Eis/NaCl in die DNA-Einzelstränge denaturiert. Nitrozellulosemembranen wurden durch Quellen in Wasser und 20x SSC (NaCl 3 Mol/l, Na-Citrat 0,3 Mol/l pH = 7) vorbehandelt und anschließend getrocknet. Nylonmembranen wurden ohne Vorbehandlung eingesetzt. Die denaturierten Nukleinsäureextrakte wurden mit einem Filtrationsgerät Minifold II der Firma Schleicher und Schüll, auf die Nitrozellulose oder Nylonmembranen aufgetragen und anschließend durch 1 h Backen bei 80°C im Vakuum oder durch 5 min UV-Vernetzung mit einem UV-Transilluminator (Nylonmembran) fixiert.

Die Nylon/Nitrozellulose-Filter wurden in einem Plastikbeutel mit 20 ml Hybridisierungs-Lösung (5x SSC; Blocking Reagens 0,5 %; N-Lauroylsarkosin, Na-Salz 0,1 %; SDS 0,02 %) verschweißt und 1 Stunde bei 68°C prähybridisiert. Die Prähybridisierungslösung wurde dann durch 2,5 ml Hybridisierungslösung (gleiche Zusammensetzung) ersetzt, die frisch denaturierte Gensonden-DNA (100 ng) enthielt. Der Hybridisierungsansatz wurde 2 Stunden bei 68°C inkubiert. Die Filter wurden anschließend 2x5 min bei Raumtemperatur mit jeweils 50 ml 2x SSC; SDS 0,1 % und dann nochmals 2x15 min bei 68°C mit 0,1x SSC 0,1 % SDS gewaschen. Die Filter wurden für die Detektion der hybridisierten DNA direkt verwendet oder luftgetrocknet für den späteren Nachweis aufbewahrt.

### Detektion der hybridisierten Nitrosomonas-DNA

Zur quantitativen Detektion der hybridisierten Nitrosomonas-DNA wurde eine immunologische Nachweisreaktion durchgeführt. Verwendet wurde ein Antikörperkonjugat mit gekoppelter alk. Phosphatase, das an die hybridisierte Digoxigenin-markierte DNA bindet. Die Farbreaktion wurde bei alkalischem pH durch Zugabe des farblosen 5-Brom-4-chlor-3-indolylphosphat und Nitroblautetrazolium gestartet. Der sich entwickelnde blaue Niederschlag wurde nach 2-12 Stunden quantitativ mit einem Densitometer der Firma Shimadzu CS430 ausgewertet. Für die Nachweisreaktion wurden folgende Puffer eingesetzt:
- Puffer 1 :: Tris/HCl 100 mmol/l; NaCl 150 mmol/l pH 7,5
- Puffer 2 :: Blocking Reagens 0,5 % in Puffer 1 lösen
- Puffer 3 :: Tris/HCl 100 mmol/l; NaCl 100 mmol/l, MgCl₂ 50 mmol/l pH 9,5
- Puffer 4 :: Tris/HCl 10 mmol/l, EDTA 1 mmol/l pH 8
Farblösung (frisch zubereitet) 45 µl NBT und 35 µl X-Phosphat wurden zu 10 ml Puffer 3 zugegeben.

Die Nitrozellulose/Nylonfilter wurden 1 min in Puffer 1 gewaschen und dann 30 min mit 100 ml Puffer 2 inkubiert und nochmals mit Puffer 1 gewaschen. Das Antikörperkonjugat wurde 1:5000 in Puffer 1 verdünnt und die Filter 30 min mit ca. 20 ml der verdünnten Antikörper-Konjugatlösung inkubiert. Ungebundenes Antikörper-Konjugat wurde durch 2x15 min Waschen mit 100 ml Puffer 1 entfernt und die Filter anschließend 2 min mit 20 ml Puffer 3 äquilibriert. Die Filter wurden dann mit 20 ml Farblösung in einem verschlossenen Plastikbeutel im Dunkeln inkubiert Die Farbintensität der einzelnen Slot Blots wurde im Vergleich zu einem mit aufgetragenen Nitrosomonas-DNA-Standard densitometrisch besfimmt.

### Spezifität der Gensonden

Die Spezifität der Gensonden wurde mit dem beschriebenen Gensonden-Test analysiert. Dazu wurden aus charakterisierten gramnegativen und grampositiven Bakterien u.a. auch Bakterien, die Halogenaromaten, Nitroaromaten, Aminoaromaten, Alkylsulfonsäuren und Arylsulfonsäuren abbauen und verschiedenen Nitrosomonas-Isolaten die Nukleinsäure extrahiert und im Dot Blot-Hybridisierungstest mit den beschriebenen Gensonden überprüft, welche Bakterienlysate zu einem positiven Hybridisierungssignal führten (Tabelle 2).

Es zeigte sich bei den durchgeführten Experimenten, daß die entwickelten Nitrosomonas-Gensonden ganz spezifisch mit allen Nitrosomonas-Lysaten hybridisierten und keine Hybridisierungssignale mit anderen Bakterienlysaten ergaben.

### Einsatz von Nitrosomonas-Gensonden zum Nachweis und zur Quantiftzierung von Nitrosomonas in Gewässern/Abwässern

Zum Nachweis und zur Quantifizierung von Nitrosomonas-Stämmen in Gewässern/Abwässern wurde zunächst die Gesamtnukleinsäure aus den abzentrifugierten Gewässer/Abwässer-Proben isoliert 50 mg Feuchtbiomasse wurden dazu mit 150 µl 0,5 M EDTA und 150 µl bidest. H₂O und 3 µl SDS 20 %ig versetzt und dann 60 Sekunden im Wasserbad bei 100°C inkubiert, anschließend sofort 1 Minute in ein Eis/Salz-Bad gestellt und dann für die 1. Phenolextraktion mit 600 µl trisgesättigtem Phenol versetzt, gemischt und 5 Minuten bei 5000 g zentrifugiert. Mit der oberen wäßrigen DNA-Phase wurde die Phenolextraktion wiederholt Phenolreste wurden durch eine anschließende Etherexnaktion entfernt.

Die Etherphase wurde abgezogen und dann mit Isopropanol die DNA gefällt und in der Tischzentrifuge bei 5000 g abzentrifugiert. Das DNA-Pellet wurde mit 70 %igem Ethanol gewaschen. Das DNA-Pellet wurde anschließend in 220 µl TE Puffer aufgenommen und wie bei der Durchführung des Gensonden-Tests beschrieben auf Nitrozellulose- oder Nylon-Membranen fixiert und dann mit den beschriebenen Gensonden hybridisiert.

Zur Quantifizierung wurde Nitrosomonas-DNA-Standard in Konzentrationen von 250 ng bis 3,5 ng entsprechend den Zellzahlen von 2,5x10⁶ bis 3,5x10⁴ Nitrosomonas-Zellen aufgetragen. Die positive Hybridisierungsreaktion wurde anhand der Farbintensität des 5-Brom-4-chlor-3-indolyl-Nitroblautetrazolium-Komplexes in einem Shimadzu CS930-Densitometer ausgemessen und im Vergleich mit den Slot Blots des Nitrosomonas-DNA-Standards die Konzentration der Nitrosomonas-spezifischen DNA bzw. der Nitrosomonas-Zellzahl in dem Probenmaterial bestimmt. Die Nachweisgrenze mit der beschriebenen Detektionsmethode lag bei 10⁵-10⁶ Nitrosomonas-Bakterien.

### Einsatz von Nitrosomonas-Gensonden zum Nachweis und zur Quantifizierung von Nitrosomonas-Stämmen in Böden

Zum Nachweis und zur Quantifizierung von Nitrosomonas-Bakterien in Böden wurde die Nukleinsäure der im Boden enthaltenen Bakterien nach der Methode von Torsvik und Marmur (4) isoliert. 10 g Boden wurden dazu mit 100 ml TE-Puffer (Tris/HCl 10 mmol/l, EDTA 1 mmol/l pH 8) versetzt, die Probe wurde gut durchmischt und anschließend der Boden vom Bakterienextrakt durch Filtration getrennt. Aus dem Filtrat wurden die Bakterien durch Zentrifugation bei 5000 g abgetrennt. Die Bakterienfraktion wurde einmal mit 100 ml 0,1 M Na₄P₂O₇ (pH7) und einmal, mit 100 ml 0,15 M NaCl, 10 mM EDTA (Saline-EDTA) gewaschen und nach der Zentrifugation bei 5000 g in 25 ml Saline-EDTA resuspendiert Durch Zugabe von 1 mg/ml Lysozym mit anschließender 30-minütiger Inkubation bei 37°C wurden die Bakterien mit Natriumdodecylsulfat (SDS) in einer Endkonzentration von 1 % lysiert. Um die noch vorhandenen Huminstoffe aus dem Boden weitgehend abzutrennen kann eine weitere Aufreinigung durch Ionenaustauschchromatographie und Hydroxylapatitchromatogrphie oder Pronase-, RNase-Behandlung und Phenolextraktion erreicht werden.

Die DNA wurde wie bei der Durchführung des Gensonden-Tests beschrieben auf Nitrocellulose- oder Nylon-Membranen fixiert und mit dem Digoxigenin-Testkit der Firma Boehringer, Mannheim, die Hybridisierungsreaktion durchgeführt Die Nitrosomonas spezifische DNA-Konzentration bzw. Zellzahl wurde über die Farbintensität des 5-Brom-4-chlor-3-indolyl-Nitroblautetrazolium-Komplexes der Slot Blots quantitativ ausgewertet.

**Tabelle 1**

| Molekulare Charakterisierung von Nitrosomonas-Klonen | | | |
|---|---|---|---|
| Klonbezeichnung | Vektor | Insert [KB] | Stamm |
| 256/15 | pBR 322 | 2.7 | Nitrosomonas |
| 256/32 | pBR 322 | 5.8 | Nitrosomonas |
| 256/36 | pBR 322 | 2.3 | Nitrosomonas |
| 256/39 | pBR 322 | 1.3 | Nitrosomonas |
| 258/21 | pBR 322 | 3.9 | Nitrosomonas |
| 258/22 | pBR 322 | 5.9 | Nitrosomonas |
| 258/23 | pBR 322 | 3.3 | Nitrosomonas |
| 258/27 | pBR 322 | 21.6 | Nitrosomonas |
| 258/29 | pBR 322 | 6.0 | Nitrosomonas |
| 258/33 | pBR 322 | 3.4 | Nitrosomonas |
| 258/34 | pBR 322 | 7.1 | Nitrosomonas |
| 258/35 | pBR 322 | 7.5 | Nitrosomonas |
| 323/9A | pSPT 19 | 6.0 | Nitrosomonas |
| 323/13B | pSPT 19 | 6.0 | Nitrosomonas |
| 322/9A | pSK | 6.0 | Nitrosomonas |
| 322/10B | pSK | 6.0 | Nitrosomonas |

Nitrosomonas-DNA kloniert als BamHI-Fragmente in dem E.coli-Vektor pBR 322 in E.coli 5K oder AG1. Die genomische DNA wurde aus dem Stamm Nitrosomonas europaea ATCC 19718 isoliert und kloniert. Die 6 kb-Gensonde aus 258/29 wurde in beiden Orientierungen in die Vektoren pSPT19 und pSK Bluescript® umkloniert.

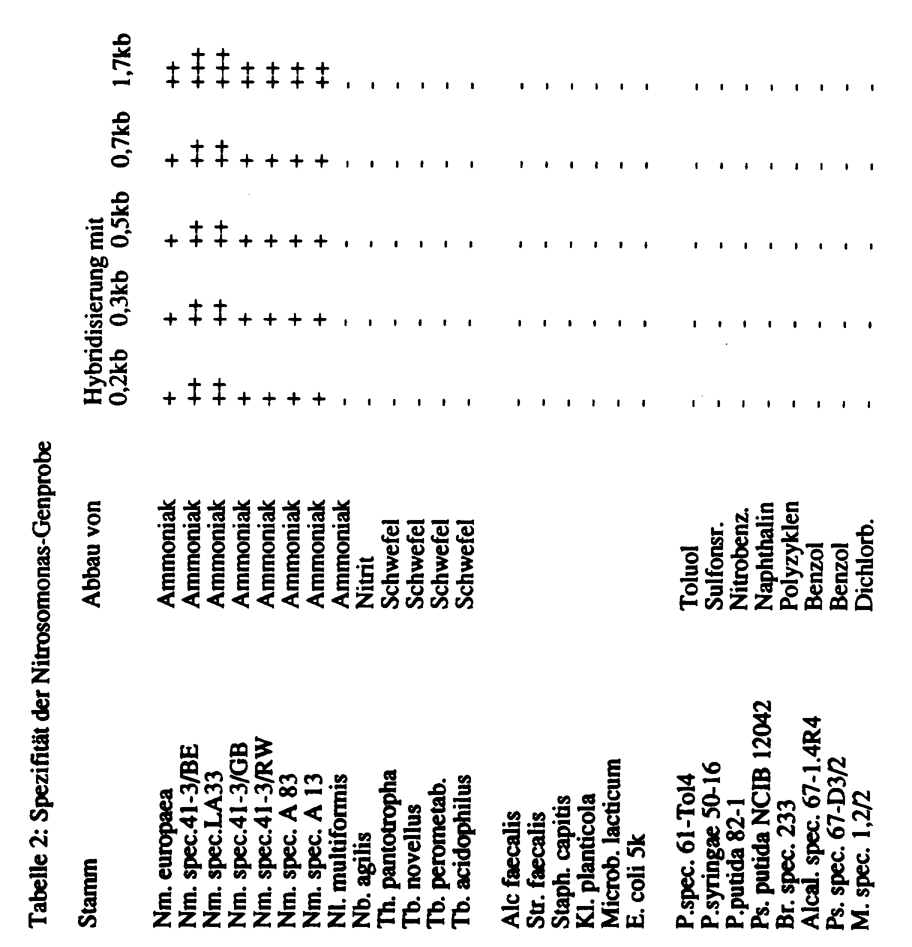

## Patentansprüche

1. Gensonde für den quantitativen Nachweis von Nitrosomonas gekennzeichnet durch 6 kb Genfragmente oder Teilen davon gemäß Abbildung 2.

2. Gensonde nach Anspruch 1 dadurch gekennzeichnet, daß sie Genfragmente umfaßt mit 1,4 kb nach Abbildung 1 und 2.

3. Gensonde nach Anspruch 1 dadurch gekennzeichnet, daß sie Genfragmente umfaßt mit 1,7 kb nach Abbildung 2 und Abbildung 3.

4. Gensonde nach Anspruch 1 dadurch gekennzeichnet, daß sie Genfragmente umfaßt mit 2,5 kb nach Abbildung 2.

5. Gensonde nach Anspruch 3 dadurch gekennzeichnet, daß sie die Nukleotidsequenz nach Abbildung 4 und 4a oder Teilen davon wie in Abbildung 1 enthält.

6. Oligonukleotidsonde dadurch gekennzeichnet, daß sie die Oligonukleotidsequenz 1 - 53 der Nukleotidsequenz von Abbildung 4 enthält.

7. Oligonukleatidsonde dadurch gekennzeichnet, daß sie die Oligonukleotidsequenz 1315 - 1365 der Nukleotidsequenz von Abbildung 4 enthält.

8. Oligonukleotidsonde dadurch gekennzeichnet, daß sie die Oligonukleotidsequenz 1610 - 1663 der Nukleotidsequenz von Abbildung 4 enthält.

9. Verfahren zum quantitativen Nachweis von Nitrosomonas dadurch gekennzeichnet, daß
a) Nukleinsäure aus Abwässern und Böden isoliert wird und zur Hybridisierung mit Gensonden nach Anspruch 1 - 8 eingesetzt wird.
b) Gensonden nach Anspruch 1 - 8 verwendet werden und mit Hilfe von bekannten Methoden mit Reportermolekülen markiert werden.
c) Eine quantitative Detektion der Nitrosomonas Nukleinsäure durch Verwendung von Gensonden nach Anspruch 1 - 8 in Hybridisierungsteste durchgeführt wird.

## Claims

1. Gene probe for quantitatively detecting Nitrosomonas, characterized by 6 kb gene fragments, or parts thereof, as shown in Figure 2.

2. Gene probe according to Claim 1, characterized in that it encompasses gene fragments of 1.4 kb as shown in Figures 1 and 2.

3. Gene probe according to Claim 1, characterized in that it encompasses gene fragments of 1.7 kb as shown in Figure 2 and Figure 3.

4. Gene probe according to Claim 1, characterized in that it encompasses gene fragments of 2.5 kb as shown in Figure 2.

5. Gene probe according to Claim 3, characterized in that it contains the nucleotide sequence shown in Figures 4 and 4a or parts thereof as in Figure 1.

6. Oligonucleotide probe, characterized in that it contains the oligonucleotide sequence 1-53 of the nucleotide sequence of Figure 4.

7. Oligonucleotide probe, characterized in that it contains the oligonucleotide sequence 1315 - 1365 of the nucleotide sequence of Figure 4.

8. Oligonucleotide probe, characterized in that it contains the oligonucleotide sequence 1610 - 1663 of the nucleotide sequence of Figure 4.

9. Process for quantitatively detecting Nitrosomonas, characterized in that
a) nucleic acid is isolated from effluent and soil samples and used for hybridizing with gene probes according to Claims 1-8.
b) gene probes according to Claims 1-8 are used and labelled with reporter molecules by means of known methods.
c) Nitrosomonas nucleic acid is quantitatively detected by using gene probes according to Claims 1-8 in hybridization tests.

## Revendications

1. Sonde génique destinée à la détermination quantitative de nitrosomonas, caractérisée par des fragments géniques de 6 kb ou des parties de ces derniers selon la figure 2.

2. Sonde génique selon la revendication 1, caractérisée en ce qu'elle comprend des fragments géniques de 1,4 kb selon les figures 1 et 2.

3. Sonde génique selon la revendication 1, caractérisée en ce qu'elle comprend des fragments géniques de 1,7 kb selon la figure 2 et la figure 3.

4. Sonde génique selon la revendication 1, caractérisée en ce qu'elle comprend des fragments géniques de 2,5 kb selon la figure 2.

5. Sonde génique selon la revendication 3, caractérisée en ce qu'elle contient la séquence de nucléotides selon les figures 4 et 4a ou des parties de cette dernière comme à la figure 1.

6. Sonde oligonucléotidique, caractérisée en ce qu'elle contient la séquence d'oligonucléotides 1-53 de la séquence de nucléotides de la figure 4.

7. Sonde oligonucléotidique, caractérisée en ce qu'elle contient la séquence d'oligonucléotides 1 315-1 365 de la séquence de nucléotides de la figure 4.

8. Sonde oligonucléotidique, caractérisée en ce qu'elle contient la séquence d'oligonucléotides 1 610-1 663 de la séquence de nucléotides de la figure 4.

9. Procédé de détermination quantitative de nitrosomonas, caractérisé en ce que
a) on isole l'acide nucléique d'eaux usées et de sols et on l'utilise pour l'hybridation avec des sondes géniques selon les revendications 1 à 8,
b) on utilise les sondes géniques selon les revendications 1 à 8 et on les marque à l'aide de procédés connus utilisant des molécules indicatrices,
c) on procède à une détection quantitative de l'acide nucléique de nitrosomonas par utilisation de sondes géniques selon les revendications 1 à 8 dans des essais d'hybridation.
